# EUROPÄISCHE PATENTSCHRIFT

(11) **EP 0 876 608 B1**
(45) Veröffentlichungstag und Bekanntmachung des Hinweises auf die Patenterteilung: **17.04.2002**
(21) Anmeldenummer: 96939933.6
(22) Anmeldetag: 27.11.1996
(51) Int. Cl.: G01N 33/487, B03C 5/02

(54) **VERFAHREN UND VORRICHTUNG ZUR ERZEUGUNG VON RESONANZERSCHEINUNGEN IN PARTIKELSUSPENSIONEN**
PROCESS AND DEVICE FOR GENERATING RESONANCE PHENOMENA IN PARTICLE SUSPENSIONS
PROCEDE ET DISPOSITIF DE PRODUCTION DE PHENOMENES DE RESONANCE DANS DES SUSPENSIONS DE PARTICULES

(30) Priorität: 27.11.1995 DE 19544127
(43) Veröffentlichungstag der Anmeldung: 11.11.1998
(73) Patentinhaber: Fuhr, Günter, 13127 Berlin (DE)
(72) Erfinder: FUHR, Günter, D-13127 Berlin (DE); GIMSA, Jan, D-10437 Berlin (DE); Müller, Torsten, D-12439 Berlin (DE); SCHNELLE, Thomas, D-10243 Berlin (DE)
(74) Vertreter: Hertz, Oliver, Dr.
(86) Internationale Anmeldenummer: EP9605244
(87) Internationale Veröffentlichungsnummer: WO9720210

(56) Entgegenhaltungen:
- DE-A- 4 400 955
- ELECTROPHORESIS, Bd. 13, 1.Januar 1992, Seiten 49-54, XP000571705 HAGEDORN R ET AL: "TRAVELING-WAVE DIELECTROPHORESIS OF MICROPARTICLES" in der Anmeldung erwähnt
- IEEE TRANSACTIONS ON INDUSTRY APPLICATIONS, Bd. 24, Nr. 2, März 1988, NEW YORK US, Seiten 217-222, XP002025002 S.MASUDA ET AL: "Movement of Blood Cells in Liquid by Nonuniform Traveling Field"
- RECORD OF THE INDUSTRY APPLICATIONS CONFERENCE (IAS), ORLANDO, OCT. 8 - 12, 1995, Bd. 2, 8.Oktober 1995, INSTITUTE OF ELECTRICAL AND ELECTRONICS ENGINEERS, Seiten 1302-1309, XP000546872 MOESNER F M ET AL: "ELECTROSTATIC DEVICES FOR PARTICLE MICRO-HANDLING"
- JOURNAL OF ELECTROSTATICS, Bd. 21, Nr. 2/03 + INDEX, September 1988, Seiten 151-191, XP000008105 ARNOLD W M ET AL: "ELECTRO-ROTATION: DEVELOPMENT OF A TECHNIQUE FOR DIELECTRIC MEASUREMENTS ON INDIVIDUAL CELLS AND PARTICLES" in der Anmeldung erwähnt

## Beschreibung

Die Erfindung betrifft ein Verfahren und eine Vorrichtung zur orts- und/oder typselektiven Steuerung der Position und/oder der Positionsänderung suspendierter Partikel in einem Multielektrodensystem mit den Merkmalen der Oberbegriffe der Patentansprüche 1 oder 9, und insbesondere planare und 3-dimensionale Mikroelektrodenanordnungen in Halbleiterchipgröße bzw. ein Verfahren, um suspendierte künstliche oder lebende Teilchen (z. B. Zellen) oder organische Teilchen von mikroskopischer Größe in Flüssigkeiten zu bewegen, zu haltern, zu vermessen oder zu sortieren. Zur individuellen Manipulation und/oder Charakterisierung derartiger Teilchen, insbesondere aber zu deren gerichteten Bewegung in einem Feldgradienten oder bewegten elektrischen Feld, werden dielektrische Polarisationskräfte genutzt, die über elektrische Wechselfelder erzeugt und durch Resonanzerscheinungen verstärkt werden.

Es sind zwei Grundprinzipien bekannt, nach denen derzeit eine elektrische Manipulation und Einzelobjektcharakterisierung durchführbar ist: 1. die Erzeugung von Feldgradienten in hochfrequenten Wechselfeldern (POHL, H.P., Dielectrophoresis, Cambridge University Press (1978)) und 2. die Applikation von rotierenden Feldern mit durchstimmbarer Rotationsfrequenz (ARNOLD, W.-M. and ZIMMERMANN,U. , Z. Naturforsch. 37c, 908, (1982)). Benachbarte, hier nicht einbezogene Gebiete wie die Elektrophorese und andere Gleichspannungstechniken lassen sich für den genannten Partikelkreis z. T. auch anwenden, sind jecoch nicht vergleichbar effektiv anwendbar.

Das erste o. a. Prinzip führt zu einer asymmetrischen Polarisation von Mikropartikeln, woraus, je nach Art der Polarisation, eine Bewegung in Richtung höherer oder niedrigerer Feldstärken erfolgt. Dieses Verhalten wird als positive bzw. negative Dielektrophorese bezeichnet (Pohl, H.P., Dielectrophoresis, Cambridge University Press, (1978)) und seit mehr als 30 Jahren zur Bewegung und der Separation suspendierter dielektrischer Körper und Zellen verwendet. In den letzten Jahren haben die dielektrophoretischen Prinzipien auf biologisch/medizinischem Gebiet eine breitere Anwendung durch die Einführung halbleitertechnologisch gefertigter Mikroelektrodensysteme erfahren (WASHIZU, M. et al., IEEE Trans. IA, 25(4), 352 (1990); SCHNELLE, Th. et al., Biochim.Biophys.Acta 1157,127 (1993)).

Das zweite o. a. Prinzip, die Applikation frequenzvariabler Rotationsfelder (in diese Kategorie fallen auch lineare Wanderfelder (HAGEDORN, R. et al., Electrophoresis 13, 49 (1992))) wird zur Charakterisierung der passiven elektrischen Eigenschaften einzelner, suspendierter Partikeln, vor allem aber von Zellen benutzt ( ARNOLD, W.-M. and ZIMMERMANN, U., Z.Naturforsch 37c, 908, (1982); FUHR, G. et al., Plant Cell Physiol. 31, 975 (1990)). Das Prinzip läßt sich wie folgt zusammenfassen: Ein Partikel befindet sich in einer zirkulären Elektrodenanordnung mit einem rotierenden Feld mit einer Geschwindigkeit von wenigen Hz bis zu einigen hundert MHz. Es verhält sich aufgrund der Lösungsreibung wie der Rotor eines dielektrischen Asynchronmotors. Im Falle von Zellen, die einen überaus komplizierten Aufbau besitzen (Zellwand, Membran, Organellen etc.), lassen die Frequenzspektren der Rotation (Partikeldrehung als Funktion der Rotationsfrequenz des Feldes) weitreichende Schlußfolgerungen über den physiologischen Zustand und die Eigenschaften einzelner Bestandteile derselben zu (ARNOLD, W.-M. and ZIMMERMANN, U., J. Electrostat. 21,151 (1988); GIMSA et al. in: W. SCHÜTT, H. KLINKMANN, I. LAMPRECHT, T. WILSON, Physical Characterization of Biological Cells, Verlag Gesundheit GmbH, Berlin 1991).

Alle elektrischen Wechselfeldverfahren nutzen Polarisationskräfte, die aus der Relaxation induzierter Ladungen resultieren. Nachteilig ist die Halbwertsbreite der dielektrischen Dispersionen, die etwa bei einer Frequenzdekade liegt (Pohl, H.P., Dielectrophoresis, Cambridge University Press, (1978); ARNOLD, W.-M. and ZIMMERMANN, U., J. Electrostat. 21,151 (1988)). Das hat zur Folge, daß die Unterscheidung oder differenzierte Bewegung verschiedener Partikeln relativ große Unterschiede in der Struktur oder den dielektrischen Eigenschaften erfordert. Ein weiteres Problem ist, daß vor allem bei kleiner werdendem Partikelradius andere Kräfte (lokale Strömung, thermische Bewegungen u.a.) an Einfluß gewinnen und bei einem Partikelradius von kleiner als einem Mikrometer die Polarisationskräfte sogar übersteigen. Bei kolloidalen Partikeln, bei denen die Polarisierbarkeit weit unter der von biologischen Zellen liegt, ist von Nachteil, daß relativ hohe Steuerspannungen (etwa 3 bis 10-fache Werte) zur Erzielung der gleichen Kraftwirkungen angewendet werden müssen.

Das ist der Grund, warum sich die beiden o. a. Prinzipien bisher nur bei relativ großen Partikeln anwenden lassen und seit langem nach einer Möglichkeit der Verstärkung der Feldeffekte gesucht wird.

Der vorliegenden Erfindung liegt die Aufgabe zugrunde, ein verbessertes Verfahren zur orts- und/oder typselektiven Steuerung der Position und/oder der Positionsänderung suspendierter Partikel in einem Multielektrodensystem und eine Vorrichtung zu dessen Ausführung anzugeben, mit denen ohne eine Erhöhung der Feldamplitude eine erhebliche Verstärkung der Polarisationskräfte bei vorher bestimmbaren Frequenzen erreicht und die natürliche Frequenzbreite der Kraftwirkungen, die sich aus den dielektrischen Dispersionen ergibt, deutlich verringert bzw. eingeengt wird.

Diese Aufgabe wird durch ein Verfahren und eine Vorrichtung mit den Merkmalen der Patentansprüche 1 oder 9 gelöst. Vorteilhafte Ausführungsformen ergeben sich aus den abhängigen Ansprüchen. Bevorzugte Verwendungen der Erfindung sind im Anspruch 20 angegeben.

Es sollen insbesondere Elektrodensysteme angegeben werden, in denen die Verstärkung der Polarisationskräfte in lokal begrenzten Bereichen (typische Abmessungen in allen drei Dimensionen einige hundert Mikrometer und kleiner) auf der Grundlage der frequenzselektiven Verstärkung der elektrischen Feldkräfte durch die Erzeugung von räumlich begrenzten Resonanzen erfolgt.

Bei den erfindungsgemäß eingesetzten Multielektrodensystemen handelt es sich zunächst um offene Schwingkreissysteme, in denen im betrachteten Frequenzintervall (≥ 100Hz) keine Resonanzerscheinungen zu erwarten wären. Im Rahmen der vorliegenden Erfindung konnte jedoch überraschenderweise festgestellt werden, daß die offenen Schwingkreissysteme durch die Partikelsuspensionen geschlossene Netzwerke bilden, in denen Resonanzen insbesondere in niedrigen Frequenzbereichen erzielbar sind.

Die Verwendung extrem miniaturisierter Elektrodensysteme (typische Abmessungen in zwei Dimensionen im µm-Bereich, in der dritten Dimension von einigen Millimetern und darunter) und deren planare bzw. dreidimensionale Anordnung bzw. Verschaltung mit kapazitiven, induktiven und ohmschen Elementen sowie die Applikation hochfrequenter elektrischer Felder (z. B. f > 10 kHz) einer Amplitude im mV- bis V-Bereich, ermöglicht die Erzeugung von lokal innerhalb der Mikrostruktur auftretenden Resonanzerscheinungen, die die Feldamplitude an diesen Stellen um ein Vielfaches erhöhen. Da die Polarisationskräfte dem Quadrat der Feldstärke proportional sind, treten vervielfachte Partikelabstoßungs- oder Anziehungskräfte (2 bis 1000-fach and mehr) auf, die zur Lösung der o. a. Aufgabenstellung genutzt werden können.

Erfindungsgemäß werden die Mikroelektroden so angeordnet, in ihren geometrischen Dimensionen gewählt oder mit Materialien beschichtet oder unterlegt, daß vorher festlegbare kapazitive, induktive und ohmsche Anteile die elektrischen Hochfrequenzeigenschaften jeder Elektrode mit dem sie umgebenden Suspensionsmedium bestimmen. Die Anteile der einzelnen Elektroden bilden nach der elektrischen Verbindung über die eingefügte Partikelsuspension Netzwerke mit Resonanzerscheinungen aus, die genau so auf einem Substrat (Glas, Silizium etc.) angeordnet werden, daß die Feldstärken dort, wo eine Partikelmanipulation oder -vermessung stattfinden soll, durch Resonanz überhöht werden, z.B. im Elektrodenzwischenraum. Die Erzeugung von Resonanzen kann durch Integration weiterer Bauelemente oder auch die externe Beschaltung mit Kondensatoren, Induktivitäten und Widerständen unterstützt bzw. bezüglich ihrer Frequenzlage verschoben und bestimmt werden. Die Kalibrierung der Resonanzfrequenz kann auch während des Betriebes erfolgen. Dieser Effekt kann sowohl für die Partikelorientierung, -bewegung, -halterung aber auch für die dielektrische Vermessung genutzt werden.

Die Resonanzeffekte können durch die Verwendung verschiedener Signalformen der Elektrodenbesteuerung selektiv, insbesondere nur für einen bestimmten spektralen Anteil des Ansteuersignals ausgenutzt werden. In Frage kommen Sinus-, Rechteck-, Dreieck- oder andere periodische und aperiodische Signalverläufe. Je nachdem, in welchem Maße die Fourierreihen zur Beschreibung dieser Signale Grund- und Oberwellen aufweisen, können Resonanzen der Grundfrequenz als auch ihrer Harmonischen ausgenutzt werden.

Die lokale Beschränkung der Resonanzen auf eine oder mehrere Bereiche von weniger als einem Kubikmillimeter wird durch die Ausführung der Elektrodenenden (zirkular, reihenförmig gegenüberliegend usw.) und die Art der.Suspensionslösung bestimmt. Aus elektronischer Sicht sind diese Anordnung als impedanzmäßig nicht definiert abgeschlossene Systeme zu bezeichnen. Wie in den Ausführungsbeispielen gezeigt wird, lassen sich jedoch Elektrodenanordnungen finden, bei denen die Abschlußimpedanz der Lösung lediglich die Amplitude der Resonanzen, nicht jedoch deren Frequenz bestimmt.

Gegenüber den in der Elektronik bekannten Resonanzeffekten sind die polarisierten Partikeln und Zellen im erfindungsgemäßen Verfahren als Probekörper für die resonanzbedingte Feldstärkeüberhöhung im Inneren des Dielektrikums (Flüssigkeit) eines Kondensators aufzufassen, 'deren Vorhandensein ihrerseits die Resonanzerscheinungen des System beeinflussen und über die Verstimmung der Resonanzfrequenz zur Partikelseparation, -sammlung und -halterung genutzt werden kann.

Besonders wirksam ist die Integration abstimmbarer kapazitiver, induktiver oder/und ohmscher Elemente, mit denen dann einzelne Elektroden individuell abgestimmt oder Resonanzen in ihrer Frequenzlage definiert und nach vorgebbaren Programmen verändert werden können. Das hat den Vorteil, daß bei gleichbleibender Elektrodenkonfiguration von außen steuerbar die Polarisationkräfte frequenzabhängig verstärkt oder gedämpft werden können. Dieser Effekt kann benutzt werden, um einzelne Partikelklassen mit gleichen oder ähnlichen dielektrischen Eigenschaften von anderen Partikelsorten abzutrennen.

Prinzipiell lassen sich die folgenden Prinzipien zur Kalibrierung und Regelung des gewünschten Resonanzeffektes anwenden:
1. Aktiver Abgleich der Schwingkreiselemente, wie Kapazitäten, Induktivitäten und Widerstände;
2. Applikation von zwei oder mehr Feldern gleicher oder entgegengesetzter Drehrichtung unterschiedlicher Frequenz;
3. Applikation von Feldern nach Pkt.2 mit jeweils unterschiedlicher aber einstellbarer Amplitude;
4. Applikation von Feldern nach Pkt.2 mit jeweils unterschiedlicher Anschaltzeit der Felder;
5. Applikation von Feldern nach Pkt.2 mit jeweils kalibrierbarer Frequenz der Felder zueinander;
6. Applikation von periodischen Signalen mit verschiedenem Oberwellenanteil;
7. Kombinationen der unter Pkt. 1 bis 6 genannten Verfahren.

In der Praxis kann die erfindungsgemäße Ausbildung von Resonanzen in Mikroelektrodensystemen nach den folgenden Prinzipien erzielt werden:

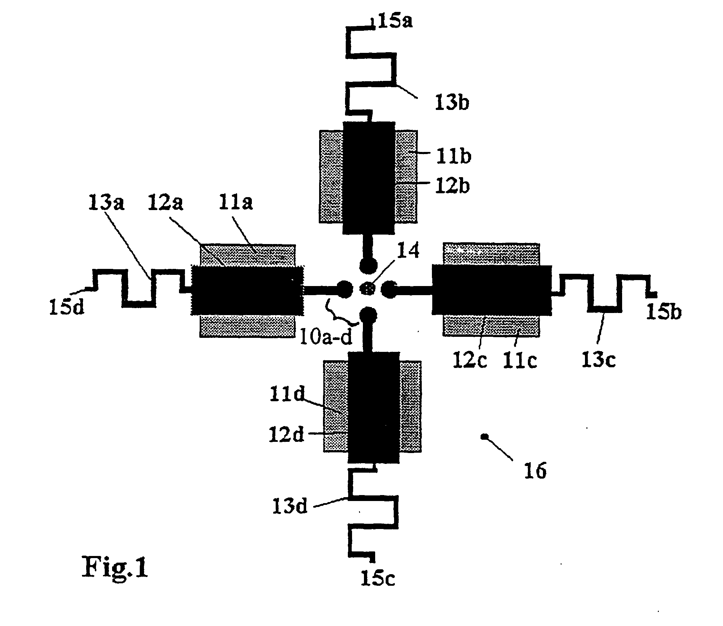

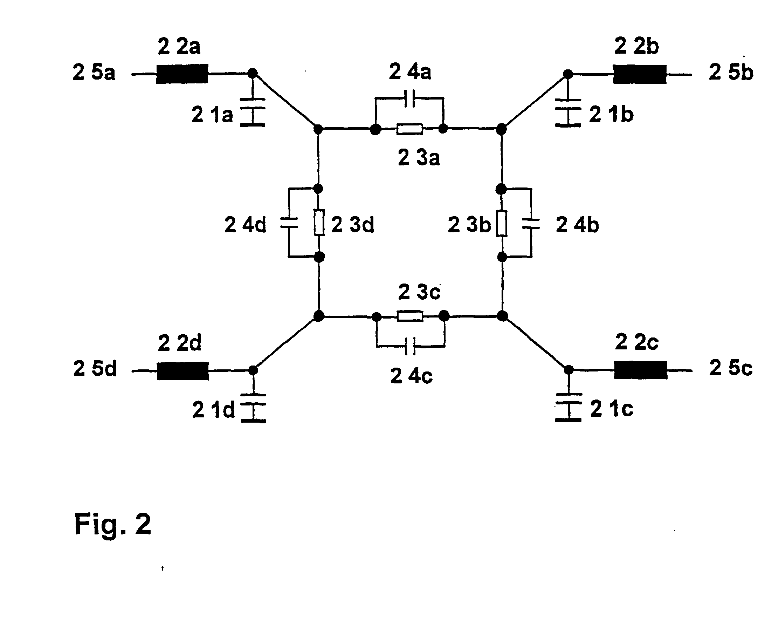

Es ist möglich, die Resonanzbedingungen über die Dimensionierung/Gestaltung der Elektroden unter Berücksichtigung der konkreten Anforderungen durch Mikrostrukturierung einzustellen. So kann z. B. das in Figur 1 gezeigte Elektrodensystem bzw. das in Figur 2 gezeigte, entsprechende Ersatzschaltbild mit üblichen Verfahren der computergestützten Netzwerkanalyse (Berechnung von Spannungsverläufen an beliebigen Orten im Netzwerk) analysiert und modelliert werden.

Ferner können die erforderlichen Eigenschaften des Elektrodensystems experimentell ermittelt werden. Dazu wird ein einem Elektrodensystem entsprechendes Netzwerk (Ersatzschaltbild) gegenständlich aufgebaut und vermessen, während ggf. zusätzliche externe Kapazitäten und/oder Induktivitäten angelegt und abgestimmt werden. Diese Verfahrensweise wird insbesondere dann bevorzugt, falls die Elektrodenzahl verhältnismäßig hoch (z. B. ≤ 8) ist.

Bei der Realisierung experimentell bestimmter Werte sind wiederum zwei Vorgehensweisen praktikabel. Erstens ist es möglich, bei der Prozessierung der Elektroden z. B. mit der Halbleitertechnologie die ermittelten Schaltelemente auf dem Chip mit zu integrieren. Dies hat die Vorteile, daß die kapazitiven/induktiven Elemente selbst Dimensionen im Mikrometerbereich einnehmen und nahezu keine Rückwirkung auf die Schaltung des Versorgungsgenerators aufweisen. Zweitens kann das Chip extern von außen beschaltet werden. Dies hat die Nachteile verringerter Gestaltungsmöglichkeiten und der über Zuleitungen nur mittelbaren Wirkung der Bauelemente im Elektrodenraum.

Schließlich ist es möglich, regelbare Bauelemente mit im Multielektrodensystem auf dem Chip zu integrieren. Zu diesen zählen insbesondere aktive Bauelemente wie Schaltdioden, Transistoren, steuerbare Kapazitätsdioden etc.. Hierbei ergibt sich der Vorteil, daß die jeweiligen Resonanzen abgestimmt durchfahren werden können und somit eine externe Computersteuerung des Systems ermöglicht wird. Eine weitere Variante in diesem Zusammenhang ist der Einsatz mechanischer Stellmittel unter Ausnutzung magnetischer oder piezoelektrischer Effekte (z. B. Feldbeeinflussung in FET).

Eine Vielzahl von Möglichkeiten bietet die halbleitertechnologische Prozessierung von planaren Elektrodenstrukturen, da hier Kapazitäten, Induktivitäten und ohmsche Anteile im Mikrometerbereich realisierbar sind. Diese Anordnung hat gegenüber einer Beschaltung von außen den Vorteil, daß sich die Netzwerke so anordnen lassen, daß die Elektroden nicht gleichmäßig, sondern in festlegbarer Weise beschaltbar sind. Weiterhin trägt dieses Prinzip wesentlich zur räumlichen Begrenzung der Resonanzerscheinung auf dem Chip oder der mikrostrukturierten Oberfläche bei.

Die erfindungsgemäße Multielektrodenanordnung kann z. B. 2, 3, 4 oder mehr Elektroden umfassen. Sie kann in Chipform in ein Keramikgehäuse mit elektrischen Anschlüssen eingefügt sein.

Bevorzugte Ausführungsbeispiele der Erfindung werden nachfolgend unter Bezugnahme auf die beiliegenden Figuren näher erläutert.

**Figur 1** veranschaulicht einen Auschnitt aus einer mikrostrukturierten Oberfläche.

Es sind vier Elektroden 10a-d, 12a-d, 13a-d (schwarz) gezeigt, die einen Zentralbereich 14 mit einem suspendierten Partikel von etwa 100 µm Durchmesser oder darunter begrenzen. Die Elektroden sind so ausgeführt, daß kapazitive und induktive Anteile bei Wechselspannungsansteuerung auftreten. Durch das Unterlegen einer verbreiterten Metallschicht 12a-d (z.B. Gold, Dicke 1 µm, Breite 100 µm) mit einem Dielektrikum 11a-d (möglichst hoher Dielektrizitätskonstante) wird ein Kondensator gebildet, der gegen Masse (hier das schematisch angegebene Substrat 16) verschaltet ist. Die mäanderförmigen Zuleitungen 13a-d) vergrößern die Induktivitäten der Elektrodenzuführungen. Die Aufeinanderzuführung der Elektroden und die elektrische Verbindung der Elektrodenenden im zentralen Bereich 14 über die Suspensionslösung führt dazu, daß die induktiven, kapazitiven und ohmschen Elektrodenanteile zu einen elektronischen Netzwerk verknüpft werden. Je nachdem, ob die Ausführung der Elektroden gleichartig oder unterschiedlich ist, können im Zentralbereich 14 der Elektroden Resonanzen erzeugt werden, die die Feldkräfte auf das Teilchen gegenüber der gleichen Ansteuerungsamplitude der Elektroden ohne die beschriebene Anordnung um ein Vielfaches steigern (in Elektrolytlösungen können das z.B. Werte zwischen >1 and 1000 oder mehr sein). Jedoch dämpft die Leitfähigkeit der Suspensionslösung die Höhe der Resonanz.

In **Figur 2** ist ein vereinfachtes Ersatzschaltbild für die in **Figur 1** gezeigte Elektrodenkonfiguration dargestellt. Es ist ersichtlich, daß die Verschaltung der Leitungskapazitäten 21a-d), der Elektrodeninduktivitäten 22a-d, der Suspensionslösungswiderstände 23a-d zwischen den Elektroden und die Kapazitäten zwischen den Elektroden 24a-d zu einem Netzwerk mit vier Eingangspolen 25a-d führt. Dieses System zeigt bei Wechselspannungsansteuerung (Pol 25a - Phase = 0 Grad, Pol 25b - Phase = 180 Grad, Pol 25c - Phase = 0 Grad, Pol 25d - Phase = 180 Grad), aber auch bei Rotationsfeldanregung (Pol 25a - Phase = 0 Grad, Pol 25b - Phase = 90 Grad, Pol 25c - Phase = 180 Grad, Pol 25d - Phase = 270 Grad) ein deutliches Resonanzverhalten. Die Frequenz kann sehr exakt über die Induktivitäten und Kapazitäten festgelegt werden.

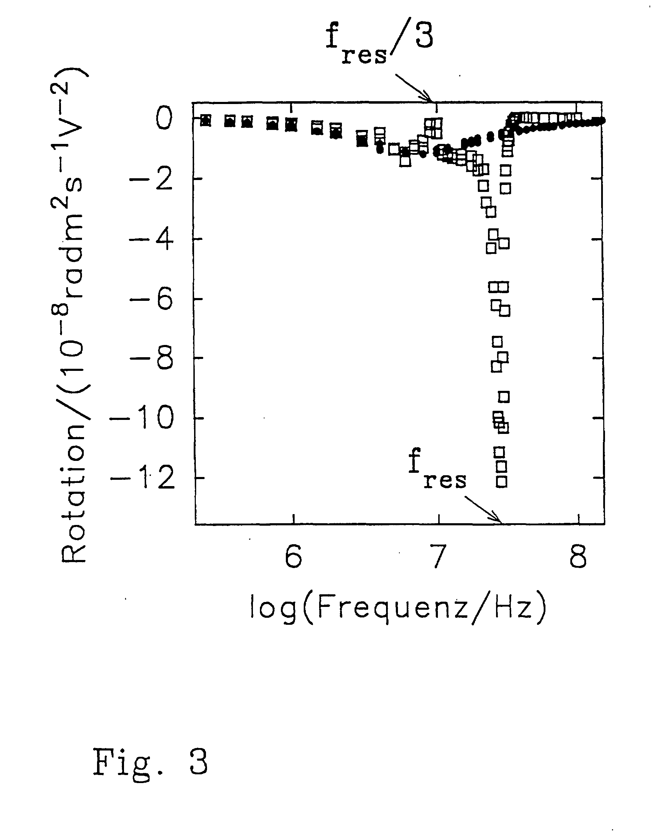

In **Figur 3** ist das Rotations-Geschwindigkeitsspektrum von Sephadexpartikeln mit 70 µm Durchmesser dargestellt. Zur Messung wurde eine Mikrokammer entsprechend **Figur 1** verwendet, die mit vier 90-Grad phasenverschobenen Rechtecksignalen mit 2 Volt Spitze-Spitze angesteuert wurde. Der Abstand zweier gegenüberliegender Elektroden betrug 100 µm und es wurde eine wässrige Lösung verwendet. Bei Ansteuerung mit einer über den gesamten Frequenzbereich konstanten Spannung wurde ein Lorenzspektrum erhalten (●). Nachdem auf dem Mikrokammerchip induktive und kapazitive Elemente integriert wurden, erhält man das durch Resonanzerscheinungen innerhalb des Zentralbereichs der Mikrokammer veränderte Spektrum (□). Deutlich ist hier die Verstärkung des Drehmomentes bei der Resonanzfrequenz , fᵣₑₛ, um mehr als den Faktor 30, sowie seine Verringerung bei einem Drittel von fᵣₑₛ zu erkennen. Diese Verringerung ist auf die Resonanzverstärkung der dritten Harmonischen bei der Meßfrequenz 1/3 fᵣₑₛ zurückzuführen. Die Ursache hierfür ist der in diesem Falle zur Grundfrequenz entgegengesetzte Drehsinn der 3. Harmonischen.

Das in **Figur 1, 2** und **3** beschriebene Quadrupolelektrodensystem eignet sich einerseits zur Zentrierung oder Halterung eines Partikels oder der Aggregatbildung aus Partikeln, insbesondere bei der Resonanzfrequenz, aber auch zur Vermessung einzelner Partikeln (z.B. Zellen) im Rotationsfeld. Die Drehgeschwindigkeit des Partikels steigt dann im Resonanzfall sehr scharf und deutlich in Abhängigkeit von der Drehfrequenz des Feldes an. Werden Rechteckfelder appliziert, treten Harmonische der Grundfrequenz und damit weitere Resonanzen in den Frequenzschichten der Kräfte, die auf die Partikeln wirken, auf.

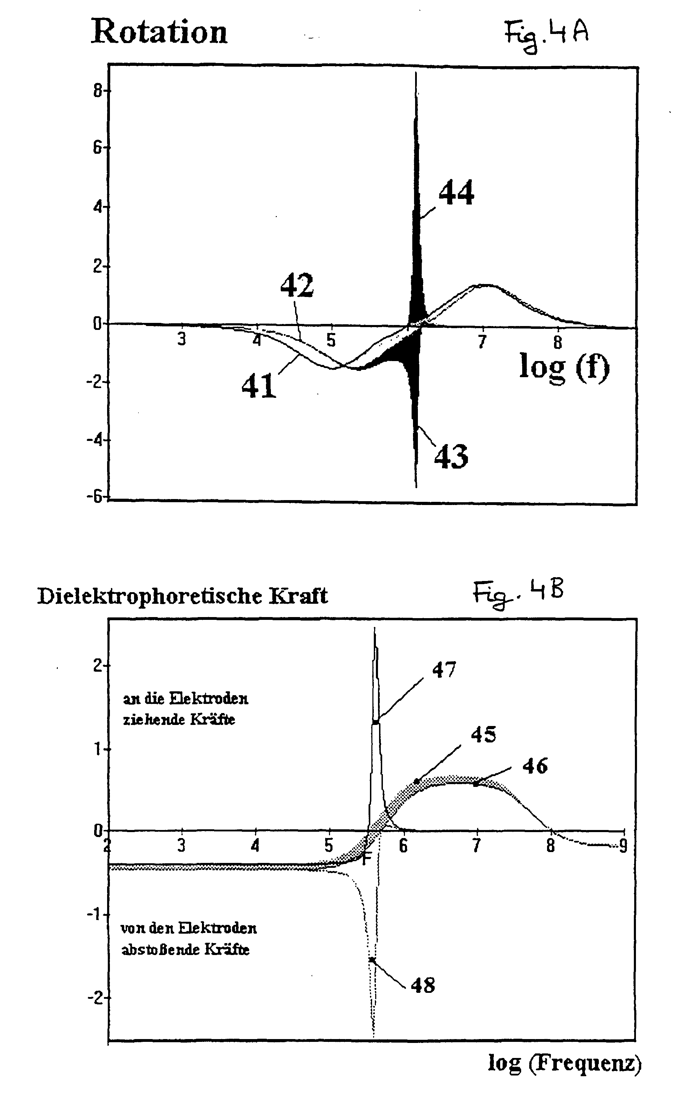

In den **Figur 4A,B** ist der Einfluß einer Resonanz auf das Rotationsverhalten von zwei dielektrischen Partikeln (hier lebende Zellen pflanzlicher oder tierischer Herkunft) geringfügig unterschiedlicher Eigenschaften (Dielektrizitätskonstante und/oder Leitfähigkeit) und damit die Unterscheidung mikroskopisch kleiner Teilchen im hochfrequenten Drehfeld dargestellt. Die Kurve 41 zeigt das Rotationsverhalten (Rot = Rotationsgeschwindigkeit als Funktion der Drehfrequenz des Feldes (f)) ohne Resonanz für das Teilchen vom Typ 1 (z.B. eine Zelle 1). Es tritt eine Rotation der Zelle sowohl entgegengesetzt zur Feldrehrichtung (niedrige Frequenzen) als auch in Richtung des Drehfeldes (hohe Frequenzen) auf. Entsprechendes ist für ein Partikel vom Typ 2 (z.B. eine Zelle 2) mit der Kurve 42 dargestellt. Legt man die Resonanz in die Nähe des Nulldurchganges der beiden Rotationsspektren, so wird der Übergang im Rotationsverhalten (Richtungswechsel) extrem steil (Kurve 42--> Kurve 43, Kurve 41--> Kurve 44). Unter diesen Bedingungen werden die Unterschiede in den dielektrischen Eigenschaften beider Teilchen also so ausgenutzt, daß sie sich schnell in unterschiedliche Drehrichtung bewegen. Der Vorteil dieser Ansteuerungsart in Verbindung mit einer Resonanz ist, daß sich Teilchen, die sich in ihren Rotationsspektrum in diesem Frequenzbereich nur geringfügig unterscheiden, dennoch nach Kurve 43, 44 entgegengesetzt drehen. Nach diesem Verhalten kann man diese Teilchensorten sehr leicht identifizieren und separieren. Das gleiche Verhalten würden Partikeln in elektrischen Wanderfelder zeigen, wobei hier die entgegengerichtete Wanderbewegung der Teilchen bei der Resonanzfrequenz direkt zum Trennen benutzt werden kann.

**Figur 4B** zeigt die gleiche Resonanzwirkung auf die dieelektrophoretischen Kräfte, die ein Partikel im Feldgradienten antreiben (45-Kraftspektrum der Zellen vom Typ 1, 46-Kraftspektrum der Zellen vom Typ 2, 47-Veränderung des Spektrums beim Auftreten einer Resonanz der Zelle vom Typ 1, 48-Veränderung des Spektrums beim Auftreten einer Resonanz der Zelle vom Typ 2).

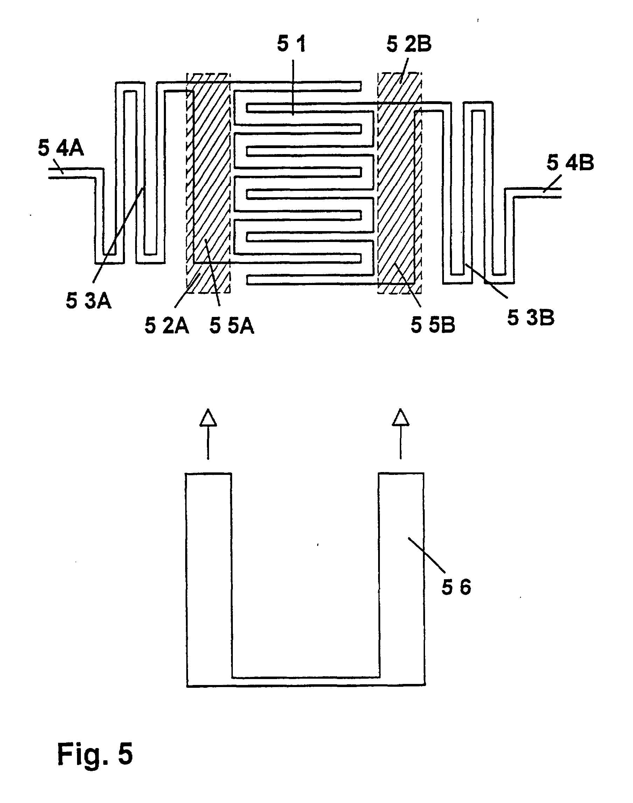

**Figur 5** stellt eine Resonanzstruktur dar, bei der der Bereich 51 durch ein elektrisches Feld zell- und partikelfrei gehalten wird. Zur Verstärkung des Feldes im Bereich 51 wird die Resonanzanordnung aus den Induktivitäten 53A und 53B und den Kapazitäten 55A und 55B ausgenutzt. Die stets vorhandene Kapazität der Kammstruktur 51 wird durch die Unterlage der Elektroden mit dem Dielektrikum 52A und 52B erhöht. Der Bereich 51 kann siebartig ausgeführt sein, so daß die Lösung durch die Struktur hindurchtreten kann, die Partikeln jedoch durch das elektrische Feld zurückgehalten werden. Das Element 56 kann als Verstimmungselement zur Justage der Resonanzfrequenz genutzt werden. Es wird auf dem Substrat prozessiert oder mikromechanisch oder auf anderem Weg über die Flächen 52 geschoben, wobei kapazitive Änderungen auftreten, die die Schwingkreise verstimmen.

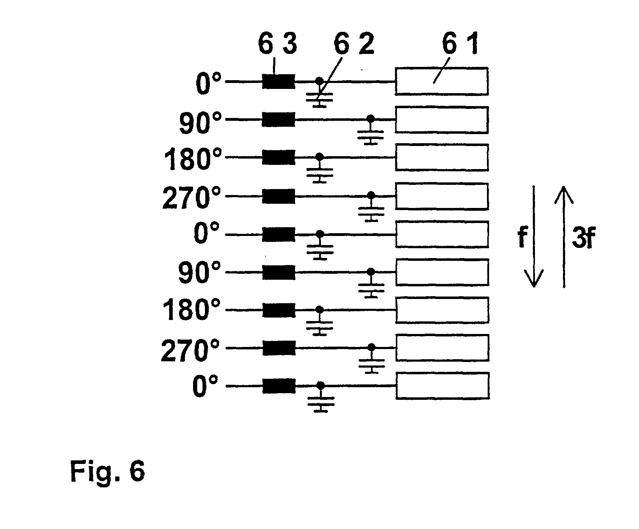

**Figur 6** stellt eine Wanderwellendielektrophoresestruktur dar. Die Partikelbewegung wird zwischen und/oder über den kammartig angeordneten Mikroelektroden (61) induziert. Wird die Struktur mit gemischten Sinussignalen (z.B. Rechtecksignalen mit dem Tastverhältnis 1:1) entsprechend der angegebenen Phasenlage angesteuert, so können die Induktivitäten 63 und Kapazitäten 62 so ausgelegt werden, daß die gewünschte Harmonische des Steuersignals durch Resonanz verstärkt wird. Dadurch ist es zum Beispiel möglich in der Kammerstruktur dieser Harmonischen die gleiche Amplitude zu verleihen wie der Grundfrequenz. Je nach Phasenbeziehung der harmonischen Frequenzen lassen sich dadurch z.B. zwei entgegengesetzt wandernde Felder eines bestimmten Frequenzverhältnisses (z.B. f und 3*f) induzieren.

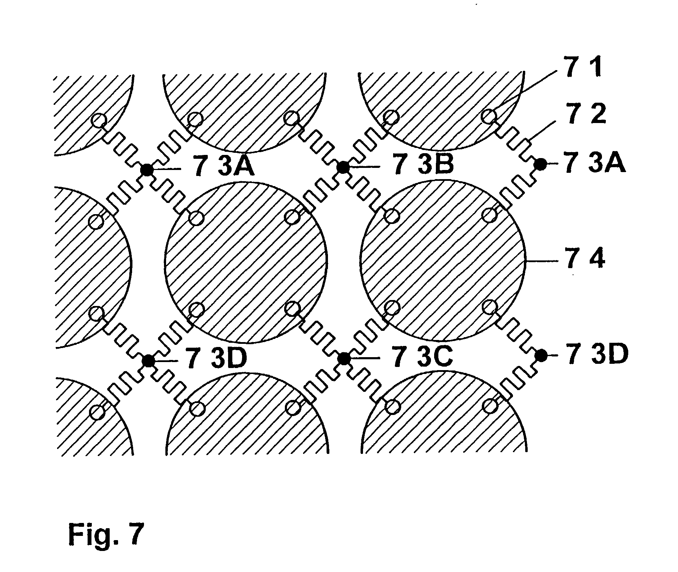

**Figur 7** zeigt eine planare Matrix-Resonanzstruktur zur Partikel- und Zellmanipulation. In dieser Struktur können gleichzeitig eine größere Menge von Partikeln oder Zellen einer Suspension manipuliert werden. Dabei steht die Suspension in den Gebieten 74 mit den Elektroden 71 galvanisch oder über eine kapazitiv wirkende Isolierschicht in elektrischem Kontakt. Zur Ansteuerung werden 4 um 90° phasenverschobene Signale an den Punkten 73A, 73B, 73C und 73D eingespeist. Durch die Beschaltung mit geeigneten Induktivitäten 72 kommt es zu einer Feldstärkeresonanzüberhöhung und einer entsprechenden Verstärkung der Feldkräfte auf einzelne Partikeln oder Zellen in den Lösungsräumen 74. Die Anzahl der in jedem Lösungsraum 74 vorhandenen Partikeln beeinflußt das Resonanzverhalten innerhalb dieses Lösungsraumes und führt z.B. zu einer Verminderung der auf weitere Partikeln wirkenden Feldkräfte. Durch dieses Verhalten der vorgeschlagenen Struktur läßt sich eine gleichmäßige Befüllung aller Lösungsräume erreichen.

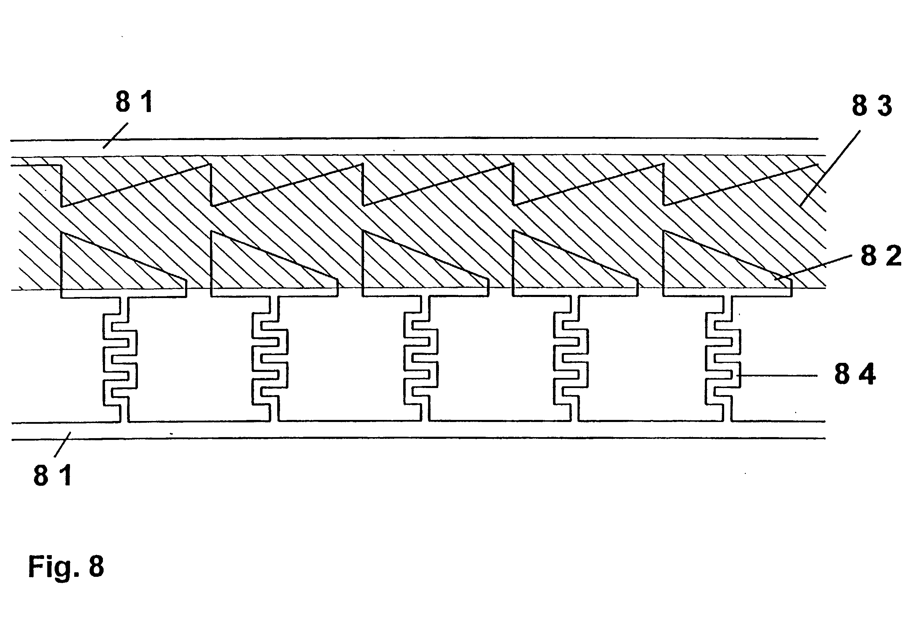

**Figur 8** zeigt eine Resonanzstruktur zur Trennung von suspendierten Partikeln oder Zellen in einem Lösungskanal 83 durch Diffusion und Feldkräfte. Die Struktur wird pulsierend oder kontinuierlich über die Anschlüsse 81 und 84 so angesteuert, daß die Partikeln oder Zellen im Wechselspiel von diffusions- und feldinduzierter Bewegung entlang des Kanals 83 wandern. Dabei werden Partikeln mit einem hohen Diffusionskoeffizienten und einer geringen positiven Dielektrophorese besonders schnell wandern. Wenn die gezeichnete Struktur so betrieben wird, daß das Suspensionsmittel die Kapazität der Elektroden 82 gegen die gemeinsame Gegenelektrode so einstellt, daß eine Resonanzüberhöhung des Feldes erfolgt, so würde die Anwesenheit von Partikeln diese Kapazität verändern und die Feldstärke an diesem Ort verringern. Dadurch gewinnt die Diffusion die Oberhand über die sammelnden Feldkräfte und ünerwünschte Häufungen der zu trennenden Partikeln oder Zellen werden vermieden.

Anwendungen der erfindungsgemäßen Steuervorrichtung liegen im Sortieren und Auftrennen von Partikelgemischen, bei medizinischen, biologischen, biotechnologischen, physikalischen und chemischen Applikationen, insbesondere im Zusammenhang mit dem Nachweis, der Charakterisierung und der Identifizierung von Zellen, Organellen, Viren und Makromolekülen, im Antrieb von dielektrischen Mikromotoren oder Mikroaktuatoren vom Rotations- oder Lineartyp, in der gerichteten Bewegung, Sortierung, Vermessung, Positionierung, Zerstörung and Modifizierung suspendierter Partikeln, in Mikromanipulationseinrichtungen, in der Assemblierung und Verkapselung von Pharmaka, in der Formung von Mikropartikeln, in der Mikrochemie (insbesondere bei der Synthese flüssiger oder fester Phasen, die erfindungsgemäß über das Resonanzprinzip gehaltert, zusammengeführt, zur Reaktion gebracht, geteilt und/oder separiert werden), oder in der Kombination mit spektroskopischen Meßverfahren (insbesondere mit der in der Veröffentlichung WO 96/16313 beschriebenen Fluoreszenz-Korrelations-Spektroskopie oder anderen, insbesondere konfokalen Fluoreszenzmeßtechniken, wie sie beispielsweise in der Veröffentlichung WO 96/13744 und der europäischen Patentanmeldung Nr. 96 116 373.0 beschrieben sind). Diese Anmeldung wurde als EP-A-0836090 veröffentlicht.

## Patentansprüche

1. Verfahren zur orts- und/oder typselektiven Steuerung der Position und/oder der Positionsänderung suspendierter Partikel in einem Multielektrodensystem unter der Wirkung von Polarisationskräften, die in den Partikeln durch elektrische Wechselfelder in dem Multielektrodensystem induziert werden, welche Partikel biologische oder synthetische Objekte mit Abmessungen umfassen, die im wesentlichen denen von biologischen Zellen oder Zellorganellen, Viren oder Makromolekülen entsprechen,
**dadurch gekennzeichnet, daß**
die Polarisationskräfte durch resonante Erhöhung oder Dämpfung der Feldstärke der elektrischen Wechselfelder bei bestimmten Frequenzen in mindestens einem lokal abgegrenzten Bereich in dem Multielektrodensystem verstärkt oder verringert werden.

2. Verfahren gemäß Anspruch 1, bei dem die resonante Feldstärkeänderung durch die externe Verstellung regelbarer Bauelemente erzielt wird, die in dem Multielektrodensystem vorgesehen sind.

3. Verfahren gemäß Anspruch 1 oder 2, bei dem die resonante Feldstärkeerhöhung durch die passiv elektrischen Eigenschaften der Partikelsuspension selektiv beeinflußt wird, wobei insbesondere durch die Anwesenheit oder Passage eines oder mehrerer Partikel in einem Bereich in dem Multielektrodensystem die elektrischen Eigenschaften der Suspension an diesem Ort so verändert werden, daß das Resonanzverhalten der Mikrostruktur verändert, festgelegt oder elektronisch vergestimmt wird, so daß die Resonanzbedingungen gegebenfalls durch die Änderung der passiven elektrischen Eigenschaften der Suspension zeitselektiv erst bei der Passage oder Anwesenheit einer bestimmten Teilchensorte, durch dieses Teilchen selbst erreicht oder beendet wird.

4. Verfahren gemäß einem der vorhergehenden Ansprüche, bei dem die resonante Feldstärkeerhöhung bei einer bestimmten Grundfrequenz der elektrischen Wechselfelder und/oder Vielfachen der Grundfrequenz erzielt wird und eine rd. 2 bis 1000-fache Verstärkung aufweist, wobei insbesondere die elektrischen Wechselfelder durch periodische Steuerspannungen einer Frequenz ≥100 Hz mit Amplituden zwischen 0,1 und 200 V erzeugt werden und die zur Felderzeugung applizierten periodischen Signale Sinus-, Dreieck-, Rechteck-, Tristateform oder eine Kombinationen aus diesen Signalarten aufweisen können, von denen ggf. bestimmte Fourierkomponenten durch die Resonanz verstärkt werden, welche Fourierkomponenten des periodischen Steuersignals gleichzeitig Feldanteile verschiedener Rotations- bzw. Translationsrichtungen erzeugen können, wobei durch die Resonanz die Amplituden der Fourierkomponenten des Feldes zueinander abgestimmt werden können.

5. Verfahren gemäß einem der vorhergehenden Ansprüche, bei dem die elektrischen Wechselfelder alternierende, rotierende oder wandernde elektrische Felder umfassen, so daß in zirkulär oder/und linear angeordneten Multielektrodenanordnungen Partikelrotationen bzw.translationen oder -positionierungen erfolgen, die nach dem Prinzip der Elektrorotation, der Dielektrophorese, der Levitation oder der Wanderwellentechnik induziert werden, wobei bestimmte Frequenzbereiche der jeweiligen Bewegungskennlinien (Spektren) durch die Resonanzerscheinungen verstärkt oder gedämpft werden.

6. Verfahren gemäß einem der vorhergehenden Ansprüche, bei dem eine Halterung der Partikeln, einzeln oder in Gruppen oder Aggregaten in Feldtrichtern oder Feldkäfigen durch die Resonanzerscheinungen verstärkt oder geschwächt wird.

7. Verfahren gemäß einem der vorhergehenden Ansprüche, bei dem die Partikel ein Gemisch aus verschiedenen Partikelarten umfassen und die Resonanzen die Bewegung eines Teils oder aller dieser Partikelarten verändern, wobei z. B. eine oder mehrere Partikeltypen bei der Resonanzfrequenz von negativer zu positiver Dielektrophorese bzw. ein Richtungswechsel bei der Partikelrotation bzw. bei der Bewegung im Wanderwellenfeld auftritt.

8. Verfahren gemäß einem der vorhergehenden Ansprüche, bei dem 2 oder mehr Feldfrequenzen moduliert, gleichzeitig über verschiedene Elektroden-Subsysteme oder auch alternierend mit gleicher oder entgegengesetzter Rotations- oder Translationsrichtung und einstellbarer Amplitude genutzt werden, indem die Resonanzfrequenz auf Bereiche des dielektrischen Partikelspektrums (Kraft als Funktion der Frequenz) gelegt wird, bei der sich die Teilchenarten unterscheiden.

9. Steuervorrichtung zur orts- und/oder typselektiven Steuerung der Position und/oder der Positionsänderung suspendierter Partikel in einem Multielektrodensystem unter der Wirkung von Polarisationskräften, die in den Partikeln durch elektrische Wechselfelder in dem Multielektrodensystem induziert werden, welche Partikel biologische oder synthetische Objekte mit Abmessungen umfassen, die im wesentlichen denen von biologischen Zellen oder Zellorganellen, Viren oder Makromolekülen entsprechen,
**dadurch gekennzeichnet, daß**
das Multielektrodensystem mit der Partikelsuspension ein elektrisches Netzwerk bildet, in dem Resonanzmittel zur Ausbildung einer resonanten Erhöhung oder Dämpfung der Feldstärke der elektrischen Wechselfelder bei bestimmten Frequenzen in mindestens einem lokal abgegrenzten Bereich in dem Multielektrodensystem vorgesehen sind.

10. Steuervorrichtung gemäß Anspruch 9, bei der die Resonanzmittel durch die kapazitive und/oder induktive Ausführung der Elektroden des Mikroelektrodensystems (z. B. Größe, Geometrie, Materialkomposition der Elektroden) gebildet werden.

11. Steuervorrichtung gemäß Anspruch 9 oder 10, bei der die Resonanzmittel durch regelbare Bauelemente gebildet werden, die in das Multielektrodensystem intergriert oder zugeschaltet sind.

12. Steuervorrichtung gemäß Anspruch 9, 10 oder 11, bei der die Resonanzmittel durch die Partikelsuspension, insbesondere die Partikel selbst, gebildet werden.

13. Steuervorrichtung gemäß einem der Ansprüche 9 bis 12, bei der die Mikroelektroden auf im wesentlichen planaren, isolierenden Trägern, z. B. aus Silizium, Glas, Keramik, Plastik, mindestens ein offenes Elektrodensystem bilden, das kapazitive, induktive und ohmsche Komponenten besitzt und über die Partikelsuspensionslösung zu mindestens einem netzwerkartigen Schwingkreissystem verbunden ist, dessen Resonanzverhalten entweder durch die Prozessierung der Mikroelektroden festgelegt oder durch das Aufbringen weiterer elektronischer Bauteile, wie Induktivitäten und/oder Kapazitäten auf dem Chip fest oder elektronisch abstimmbar eingestellt ist.

14. Steuervorrichtung gemäß Anspruch 13, bei der das Elektrodensystem in Form von mäander- und/oder schleifenförmigen Ausbildungen gestaltet ist und Unterlegungen bzw. Überdeckungen von diesen mit isolierenden und/oder dielektrischen Schichten aufweist, oder bei der die Mikroelektroden durch paarige, symmetrische, zirkular und/oder lineare Anordnungen Netzwerke mit einem veränderten Einfluß von kapazitiven und/oder induktiven Elementen bilden, wobei sich ggf. einzelne Resonanzräume gegenseitig beeinflussen.

15. Steuervorrichtung gemäß einem der Ansprüche 9 bis 14, bei der die Mikroelektrodenanordnungen typische Gap-Abmessungen von 10 nm bis zu einigen 100 µm aufweisen und in denen rotierende oder alternierende elektrische Felder erzeugt werden.

16. Steuervorrichtung gemäß einem der Ansprüche 9 bis 15, bei der die Mikroelektrodenanordnungen 3-dimensionale Strukturen oder Mehrebenenstrukturen auf einem Substrat aufweisen, das aus Glas, Halbleitermaterial, Plastik oder Keramik besteht.

17. Steuervorrichtung gemäß einem der Ansprüche 9 bis 16, bei der das Substrat Strukturierungen, passive Bauteile, Oberflächen mit Kanälen, Wällen, Vertiefungen, Durchbrüchen oder Barrieren, und/oder mikromechanische Elemente, wie Klappen, Membranen, verschiebbare Körper oder bewegliche Arme zur Verstimmung der vorhandenen Schwingkreise aufweist, oder bei der die Endstufen oder der gesamte Hochfrequenzgenerator zur Erzeugung der Elektrodensignale oder/und zur Ansteuerung der Bauteile zur Regelung der Resonanzfrequenzen auf dem Substrat integriert sind.

18. Steuervorrichtung gemäß einem der Ansprüche 9 bis 17, bei der mehrere Mikroelektrodensysteme nebeneinander, hintereinander, versetzt, einander entgegengerichtet, kaskadenförmig, ringartig oder stapelartig angeordnet sind.

19. Steuervorrichtung gemäß einem der Ansprüche 9 bis 18, bei der Bauelemente vorgesehen sind, mit denen die Regelung der Resonanzfrequenzen erfolgt und die von einem vorgebbaren Regelprogramm steuerbar sind, welche Bauelemente durch aktive Bauelemente wie z. B. Kapazitätsdioden, Feldeffekttransistoren und geregelte Induktivitäten gebildet werden, wobei die Veränderungen in den Resonanzerscheinungen auch durch Zu- oder Abschalten oder Überbrücken von Schwingkreisbauteilen erzeugbar sind.

20. Verwendung einer Steuervorrichtung gemäß einem der Ansprüche 9 bis 19,
+ zum Sortieren und Auftrennen von Partikelgemischen,
+ für medizinische, biologische, biotechnologische, physikalische oder chemische Anwendungen im Zusammenhang mit dem Nachweis und der Identifizierung von Zellen, Organellen, Viren oder Makromoleküle,
+ zum Antrieb von dielektrischen Mikromotoren oder Mikroaktuatoren vom Rotations- oder Lineartyp,
+ zur gerichteten Bewegung, Sortierung, Vermessung und Positionierung suspendierter Partikeln, oder
+ als Bestandteil einer Mikromanipulationseinrichtung.

## Claims

1. A method of positionally selective and/or type selective control of the position and/or the change in position of suspended particles in a multi-electrode system, under the action of polarisation forces, which are induced in the particles by electric alternating fields in the multi-electrode system, which particles comprise biological or synthetic objects with dimensions which correspond substantially to those of biological cells or cell organelles, viruses or macro-molecules, **characterized in that** the polarisation forces are increased or reduced in at least one locally restricted region in the multi-electrode system by resonant enhancement or attenuation of the field strength of the electric alternating fields at specific frequencies.

2. A method according to claim 1, in which the resonant field strength alteration is achieved by the external adjustment of components which can be regulated and are provided in the multi-electrode system.

3. A method according to claim 1 or 2, in which the resonant field strength enhancement is selectively affected by the passive electrical properties of the particle suspension, wherein the electrical properties in a region in the multi-electrode system are so altered at this place, in particular by the presence or passage of one or more particles, that the resonance behaviour of the microstructure changes, is determined or electronically detuned, so that the resonance conditions are only achieved or terminated time-selectively through a specific kind of particles by the passage or presence of these particles themselves, optionally through the alteration of the passive electrical properties of the suspension.

4. A method according to any of the preceding claims, in which the resonant field strength enhancement is achieved at a specific fundamental frequency of the electric alternating field and/or multiples of the fundamental frequency and has an amplification of around 2 to 1000 times, wherein in particular the electric alternating fields are generated by periodic control voltages of a frequency ≥ 100 Hz with amplitudes between 0.1 and 200 V and the periodic signals applied for generating the fields can have sinusoidal, triangular, rectangular, tri-state form or a combination of these kinds of signal, of which optionally specific Fourier components are amplified by the resonance, which Fourier components of the periodic control signals can simultaneously create field components of different rotational or translational directions, wherein the amplitudes of the Fourier components of the field can be synchronised relative to one another by the resonance.

5. A method according to any of the preceding claims, in which the electric alternating fields comprise rotating or travelling electric fields, so that particle rotations or translations or changes in position are effected in circularly and/or linearly arranged multi-electrode arrangements and are induced according to the principle of electro-rotation, dielectrophoresis, levitation or travelling wave technology, wherein specific frequency ranges of the respective movement characteristics (spectra) are amplified or attenuated by the resonance effects.

6. A method according to any of the preceding claims, in which retention of the particles, individually or in groups or aggregates in field funnels or field cages is amplified or attenuated by the resonance effects.

7. A method according to any of the preceding claims, in which the particles comprise a mixture of different kinds of particles and the resonances alter the movement of part or all of these kinds of particles, wherein for example one or more particle types goes from negative to positive dielectrophoresis or a change of direction of the particle rotation or the movement in the travelling wave field occurs.

8. A method according to any of the preceding claims, in which 2 or more field frequencies are modulated, simultaneously by different electrode sub-systems or alternately with the same or opposite direction of rotation or translation and adjustable amplitudes are used, in which the resonance frequency is imposed on ranges of the dielectric particle spectrum (force as a function of frequency) at which the particle types differ.

9. Control apparatus for positionally selective and/or type selective control of the position and/or the change in position of suspended particles in a multi-electrode system, under the action of polarisation forces, which are induced in the particles by electric alternating fields in the multi-electrode system, which particles comprise biological or synthetic objects with dimensions which correspond substantially to those of biological cells or cell organelles, viruses or macro-molecules, **characterized in that** the multi-electrode system forms an electrical network with the particle suspensions, in which resonance means are provided for creating a resonant enhancement or attenuation of the field strengths of the electric alternating fields at specific frequencies in at least one locally restricted region in the multi-electrode system.

10. Control apparatus according to claim 9, in which the resonance means are formed by the capacitive and/or inductive design of the electrodes of the macro-electrode system (e.g. size, geometry, material composition of the electrodes).

11. Control apparatus according to claim 9 or 10, in which the resonance means are formed by components which can be regulated and are integrated in or can be switched into the multi-electrode system.

12. Control apparatus according to claim 9, 10 or 11, in which the resonance means are formed by the particle suspension, especially the particles themselves.

13. Control apparatus according to any of claims 9 to 12, in which the micro-electrodes on substantially planar, insulating substrates, e.g. of silicon, glass, ceramic, plastics material, form at least one open electrode system which has capacitive, inductive or ohmic components and is connected through the particle suspension solution into at least one oscillatory system like a network, whose resonant behaviour is determined either by the processing of the micro-electrodes or is adjusted fixedly or able to be tuned electronically by the application of further electronic components, such as inductances and/or capacitances on the chip.

14. Control apparatus according to claim 13, in which the electrode system is designed in the form of meandering and/or loop-shaped arrangements and has underlays or overlays thereof with insulating and/or dielectric layers, or in which the micro-electrodes form networks with a modified effect of capacitive and/or inductive elements, through paired, symmetrical, circular and/or linear arrangements, wherein individual resonance zones optionally affect each other reciprocally.

15. Control apparatus according to any of claims 9 to 14, in which the micro-electrode arrangements have typical gap dimensions from 10 nm up to some 100 µm and in which rotating or alternating electric fields are generated.

16. Control apparatus according to any of claims 9 to 15, in which the micro-electrode arrangements comprise three-dimensional structures or multi-plane structures on a substrate, which consists of glass, semiconductor material, plastics material or ceramic.

17. Control apparatus according to any of claims 9 to 16, in which the substrate has structuring, passive components, surfaces with channels, bulwarks, depressions, perforations or barriers, and/or micro-mechanical elements such as flaps, membranes, movable bodies or movable arms for de-tuning the existing oscillatory circuit, or on which the final stages or the whole high frequency generator for generating the electrode signals or/and for controlling the components for regulation of the resonance frequencies are integrated on the substrate.

18. Control apparatus according to any of claims 9 to 17, in which a plurality of micro-electrode systems are arranged beside one another, behind one another, offset, opposed to one another, in cascade form, ring-like or stack-like.

19. Control apparatus according to any of claims 9 to 18, in which components are provided with which the regulation of the resonance frequencies is effected and which can be controlled by a predetermined control program, which components are formed by active components, such as variable capacitance diodes, field-effect transistors and regulated inductances for example, wherein the changes in the resonance effects can also be created by switching on or off or bridging oscillatory circuit components.

20. Use of a control apparatus according to any of claims 9 to 19:
+ for sorting out and separating particle mixtures,
+ for medical, biological, bio-technological, physical or chemical applications in connection with the detection and identification of cells, organelles, viruses or macro-molecules,
+ to drive dielectric micro-motors or micro-actuators of the rotary or linear type,
+ for directed movement, sorting, measurement and positioning of suspended particles, or
+ as a component of a micro-manipulation device.

## Revendications

1. Méthode pour le contrôle sélectif en lieu et en type de la position et/ou du changement de position de particules en suspension dans un système multiélectrodes sous l'effet de forces de polarisation, qui sont induites dans les particules par des champs alternatifs électriques dans le système multiélectrodes, lesquelles particules comprennent des objets biologiques ou synthétiques avec des dimensions qui correspondent sensiblement à celles de cellules biologiques ou d'organelles cellulaires, de virus ou de macromolécules,
**caractérisée en ce que** les forces de polarisation sont renforcées ou réduites par une augmentation résonante ou une atténuation de l'intensité de champ des champs alternatifs électriques pour des fréquences définies dans au moins une plage délimitée localement dans le système multiélectrodes.

2. Méthode selon la revendication 1, avec laquelle la variation résonante de l'intensité du champ est obtenue par l'ajustage externe de composants réglables qui sont prévus dans le système multiélectrodes.

3. Méthode selon la revendication 1 ou 2, avec laquelle l'augmentation résonante de l'intensité du champ est influencée de façon sélective par les propriétés électriques passives de la suspension de particules, moyennant quoi les propriétés électriques de la suspension sont modifiées en cet endroit en particulier par la présence ou le passage d'une ou de plusieurs particules dans une zone dans le système multiélectrodes de telle sorte que le comportement à la résonance de la microstructure est modifié, déterminé ou harmonisé au plan électronique, ce qui fait que les conditions de résonance sont obtenues ou achevées éventuellement par la modification des propriétés électriques passives de la suspension de façon sélective dans le temps seulement lors du passage ou de la présence d'une certaine sorte de particule, par cette particule même.

4. Méthode selon l'une quelconque des revendications précédentes, avec laquelle l'augmentation résonante de l'intensité du champ est obtenue avec une certaine fréquence de base des champs alternatifs électriques et/ou un multiple de la fréquence de base et présente une intensification d'environ 2 à 100 fois, les champs alternatifs électriques en particulier étant générés par des tensions de commande périodiques d'une fréquence supérieure ou égale à 100 HZ avec des amplitudes comprises entre 0,1 et 200 v et les signaux périodiques appliqués pour la génération du champ pouvant présenter une forme sinusoïdale, triangulaire, rectangulaire, trois états, ou une combinaison de ces types de signaux, dont éventuellement certaines composantes de Fourier sont renforcées par la résonance, lesquelles composantes de Fourier du signal de commande périodique peuvent générer en même temps des parties de champ avec différents sens de rotation ou de translation, moyennant quoi les amplitudes des composantes de Fourier du champ peuvent être harmonisées entre elles par la résonance.

5. Méthode selon l'une quelconque des revendications précédentes, avec laquelle les champs alternatifs électriques comprennent des champs électriques alternatifs, rotatifs ou mobiles, de sorte qu'il survient dans des agencements multiélectrodes disposés de façon circulaire ou linéaire des rotations et des translations ou positionnements de particules qui sont induits selon le principe de l'électrorotation, de la diélectrophorèse, de la lévitation ou de la technique d'ondes progressives, certaines plages de fréquence des courbes caractéristiques de mouvement respectives (spectres) étant renforcées ou atténuées par les phénomènes de résonance.

6. Méthode selon l'une quelconque des revendications précédentes, avec laquelle une fixation des particules est renforcée ou atténuée par les phénomènes de résonance individuellement ou en groupes ou agrégats dans des entonnoirs de champ ou des cages de champ.

7. Méthode selon l'une quelconque des revendications précédentes, avec laquelle les particules comprennent un mélange de différents types de particules et les résonances modifient le déplacement d'une partie ou de tous ces types de particules, moyennant quoi par exemple un ou plusieurs types de particules apparaissent lors de la fréquence de résonance d'électrophorèse négative en électrophorèse positive ou bien il survient un changement de direction lors de la rotation des particules ou lors du déplacement dans le champ d'ondes progressives.

8. Méthode selon l'une quelconque des revendications précédentes, avec laquelle deux fréquences de champ ou plus sont modulées et utilisées simultanément par différents sous-systèmes à électrodes ou également en alternance avec un sens de rotation ou translation identique ou opposé et une amplitude réglable en appliquant la fréquence de résonance à des zones du spectre de particule diélectrique (force en fonction de la fréquence), fréquence à laquelle les types de particules se différencient.

9. Dispositif de commande pour le contrôle sélectif en lieu et en type de la position et/ou du changement de position de particules en suspension dans un système multiélectrodes sous l'effet de forces de polarisation qui sont induites dans les particules par des champs alternatifs électriques dans le système multiélectrodes, lesquels particules comprennent des objets biologiques ou synthétiques avec des dimensions qui correspondent sensiblement à celles de cellules biologiques ou d'organelles cellulaires, de virus ou de macromolécules, **caractérisé en ce que** le système multiélectrodes forme avec la suspension de particules un réseau électrique dans lequel des moyens de résonance sont prévus pour réaliser une élévation résonante ou une atténuation de l'intensité de champ des champs alternatifs électriques pour certaines fréquences dans au moins une zone délimitée localement dans le système multiélectrodes.

10. Dispositif de commande selon la revendication 9, sur lequel les moyens de résonance sont constitués par la réalisation capacitive et/ou inductive des électrodes du système à microélectrodes (par exemple grandeur, géométrie, composition du matériau des électrodes).

11. Dispositif de commande selon la revendication 9 ou 10, sur lequel les moyens de résonance sont formés par des composants réglables qui sont intégrés dans le système multiélectrodes ou sont mis en circuit.

12. Dispositif de commande selon la revendication 9, 10 ou 11, sur lequel les moyens de résonance sont constitués par la suspension de particules, en particulier les particules mêmes.

13. Dispositif de commande selon l'une quelconque des revendications 9 à 12, sur lequel les microélectrodes sur des supports isolants sensiblement planaires et isolants, par exemple à base de silicium, verre, céramique, plastique, forment au moins un système d'électrodes ouvert, qui présente des composants capacitifs, inductifs ou ohmiques et est relié par le biais de la solution de suspension de particules à au moins un système de circuit oscillant de type réseau, dont le comportement à la résonance est défini par le processus des microélectrodes ou est réglé de façon fixe ou de façon accordable au plan électronique par l'application d'autres composants électroniques tels que des inductances et/ou des condensateurs sur la puce.

14. Dispositif de commande selon la revendication 13, sur lequel le système d'électrodes est conçu sous la forme de réalisations en méandres et/ou boucles et présente des parties passant au-dessous ou au-dessus de ces réalisations avec des couches isolantes et/ou diélélectriques, ou sur lequel les microélectrodes forment au moyen d'agencements par paires, symétriques, circulaires et/ou linéaires des réseaux avec une influence modifiée d'éléments capacitifs et/ou inductifs, moyennant quoi des espaces de résonance individuels s'influencent éventuellement réciproquement.

15. Dispositif de commande selon l'une quelconque des revendications 9 à 14, sur lequel les agencements à microélectrodes présentent des dimensions Gap caractéristiques de 10 nm jusqu'à quelques centaines de µm et dans lesquels sont générés des champs électriques rotatifs ou alternatifs.

16. Dispositif de commande selon l'une quelconque des revendications 9 à 15, sur lequel les agencements à microélectrodes présentent des structures en trois dimensions ou des structures multiniveau sur un substrat, qui est en en verre, matériau semiconducteur, plastique ou céramique.

17. Dispositif de commande selon l'une quelconque des revendications 9 à 16, **caractérisé en ce que** le substrat présente des structurations, des composants passifs, des surfaces avec des canaux, des ondulations, des cavités, des passages ou des barrières, et/ou des éléments de micromécanique tels que des clapets, des membranes, des corps coulissants ou des bras mobiles pour le désaccord des circuits oscillants présents, ou sur lequel les étages de sortie ou l'ensemble du générateur de haute fréquence pour la génération des signaux d'électrodes et/ou pour la commande d'amorçage des composants pour le réglage des fréquences de résonance sont intégrés sur le substrat.

18. Dispositif de commande selon l'une quelconque des revendications 9 à 17, sur lequel plusieurs systèmes à microélectrodes sont disposés les uns à côtés des autres, les uns derrière les autres, décalés, orientés dans le sens contraire, en forme de cascade, d'anneau ou de pile.

19. Dispositif de commande selon l'une quelconque des revendications 9 à 18, sur lequel il est prévu des composants avec lesquels s'effectue le réglage des fréquences de résonance et qui peuvent être contrôlés par un programme de réglage prédéfinissable, lesquels composants sont formés par des composants actifs tels que des diodes à capacité variable, des transistors à effet de champ et des inductances régulées, les modifications dans les phénomènes de résonance pouvant être générées également par la mise en circuit ou hors circuit ou le shuntage de composants de circuit oscillant.

20. Utilisation d'un dispositif de commande selon l'une quelconque des revendications 9 à 19,
- pour le triage et la séparation de mélanges de particules,
- pour des applications médicales, biologiques, biotechnologiques, physiques ou chimiques en rapport avec le décèlement et l'identification de cellules, d'organelles, de virus ou de macromolécules,
- pour l'entraînement de micromoteurs diélectriques ou de microactionneurs de type rotatif ou linéaire,
- pour le déplacement orienté, le triage, le mesurage et le positionnement de particules en suspension, ou
- comme composant d'un système de micromanipulation.
